(19) Europäisches Patentamt
European Patent Office
Office européen des brevets

(11) EP 2 891 482 A1

# (12) EUROPEAN PATENT APPLICATION

published in accordance with Art. 153(4) EPC

(43) Date of publication:
**08.07.2015 Bulletin 2015/28**

(21) Application number: **13832257.3**

(22) Date of filing: **30.08.2013**

(51) Int Cl.:
***A61K 8/81*** *(2006.01)* ***A61K 8/02*** *(2006.01)*
***A61K 8/46*** *(2006.01)* ***A61K 8/73*** *(2006.01)*
***A61Q 5/06*** *(2006.01)*

(86) International application number:
**PCT/JP2013/073252**

(87) International publication number:
**WO 2014/034824 (06.03.2014 Gazette 2014/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **30.08.2012 JP 2012190228**

(71) Applicant: **Kao Corporation**
**Chuo-ku**
**Tokyo 103-8210 (JP)**

(72) Inventor: **TANIMURA, Tadashi**
**Tokyo 131-8501 (JP)**

(74) Representative: **Hoffmann Eitle**
**Patent- und Rechtsanwälte PartmbB**
**Arabellastraße 30**
**81925 München (DE)**

## (54) HAIR COSMETIC

(57) A hair cosmetic composition comprising the following components
(A) to (C) :
(A) A polymer or copolymer having the structural units represented by the general formula (1):

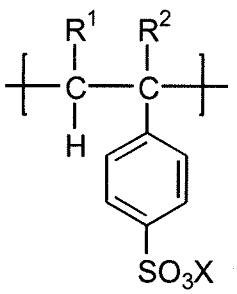

(1)

(B) cyclodextrin; and
(C) 50 to 95% by mass of water.

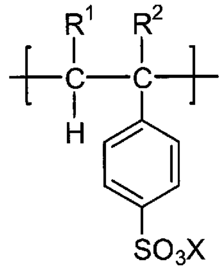

(1)

EP 2 891 482 A1

**Description**

Field of the Invention

The present invention relates to a hair cosmetic composition.

Background of the Invention

Example of hair styling cosmetic composition includes head hair cosmetics such as hair styling mist, hair styling foam, hair gel, hair cream, and hair wax, and eyelash cosmetics such as mascara. These hair styling cosmetic compositions contain film-forming resins for imparting hair styling property. There are various film-forming resins. Usually, a film-forming resin is dissolved in a solvent such as ethanol, thereby stably being contained in a hair styling cosmetic composition.

In recent years, the reduction of the use of organic solvents such as ethanol is preferred, from the viewpoints of environmental considerations.

Patent Document 1 proposes the use of cyclodextrin in an aqueous hair styling composition containing a reduced amount of organic solvent, thereby improving solubility of the hair styling polymer.

Patent Document 2 discloses a hair cosmetic composition containing a vinylpyrrolidone polymer and a water-soluble dextrin. This Document discloses that this composition improves the hair set retentivity without impairing the stability of the system, and will not impair hair washability.

Citation List

Patent documents

Patent document 1: WO 2002/38113 A
Patent Document 2: JP 2005-120045 A

Summary of the Invention

The present invention provides a hair cosmetic composition comprising following components (A) to (C):

(A) a polymer or copolymer having structural units represented by the general formula (1);

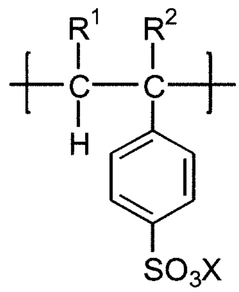

(1)

wherein $R^1$ and $R^2$ independently represent a hydrogen atom, a methyl group, or a carboxy group, and X represents H, Na, K, Li, $NH_4$, or $CH_2CH_3$;
(B) cyclodextrin; and
(C) 50 to 95% by mass of water.

Detailed Description of the Invention

A drawback when an aqueous hair cosmetic composition is applied to the hair is penetration of moisture into hair, which causes dangling of hair (folding of hair), and deformation of the hair set. Another drawback is that the hair setting ability after setting the hair cannot be maintained for a long time in high humidity, whereby the hair setting property can decrease. For example, when a vinylpyrrolidone polymer is used as the hair cosmetic composition in Patent Document

2, the hair set retentivity in high humidity was insufficient.

In addition, when the combination of an acrylic set polymer and a cyclodextrin is used as in Patent Document 1, bubbles are formed in the nozzle after spraying the hair cosmetic composition, i.e. so-called "after draw", and the bubbles thus formed can adhere to hair during spraying to cause flaking.

The present invention relates to an aqueous hair cosmetic composition which prevents hair dangling after application, and retains hair setting property for a long time even in high humidity.

[(A): Polymer or copolymer]

The hair cosmetic composition of the present invention comprises a polymer or copolymer having the structural units represented by the general formula (1) as component (A):

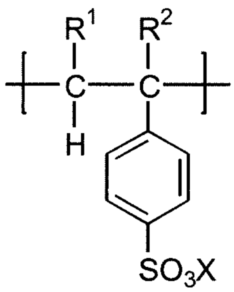

(1)

wherein $R^1$ and $R^2$ each independently represent a hydrogen atom, a methyl group, or a carboxy group, X represents H, Na, K, Li, $NH_4$, or $CH_2CH_3$;

In addition to the structural units represented by the general formula (1), the structural units represented by the general formula (2) may be contained:

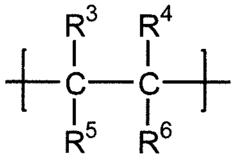

(2)

wherein $R^3$, $R^4$ and $R^5$ independently represent a hydrogen atom, a methyl group, or a carboxy group, $R^6$ represents an optionally substituted phenyl group, a substituent represented by $-COOR^7$ (wherein $R^7$ represents a hydrogen atom, Na, K, Li, or an optionally substituted hydrocarbon group having 1 to 24 carbon atoms), a substituent represented by $-COR^8$ (wherein $R^8$ represents an optionally substituted hydrocarbon group having 1 to 24 carbon atoms), or a substituent represented by $-COO(AO)_nR^9$ (wherein AO represents an ethylene oxide group and/or a propylene oxide group, n represents a numeral of 1 to 15 on average, and $R^9$ represents a hydrogen atom or an optionally substituted hydrocarbon group having 1 to 5 carbon atoms).

Among them, $R^3$, $R^4$ and $R^5$ are preferably independently a hydrogen atom or a methyl group, $R^6$ is preferably a phenyl group or a substituent represented by $-COOR^7$ (wherein $R^7$ is hydrogen atom or Na).

The polymer or copolymer of the component (A) contains the structural units represented by the general formula (1), in the amount of preferably 10 to 100% by mass, more preferably 20 to 100% by mass, more preferably 30 to 100% by mass, more preferably 40 to 100% by mass, more preferably 50 to 100% by mass, more preferably 60 to 100% by mass, more preferably 70 to 100% by mass, more preferably 80 to 100% by mass, and more preferably 90 to 100% by mass in one molecule, from the viewpoint of preventing hair dangling after application, maintaining hair setting property, and preventing the occurrence of after draw during spraying when cyclodextrin as the component (B) is used in combination.

Specific examples of the polymer or copolymer of the component (A) include sodium polystyrene sulfonate (e.g. PS-1 having a weight average molecular weight of 10,000 to 30,000, PS-5 having a weight average molecular weight of 50,000 to 100,000, PS-15 having a weight average molecular weight of 150,000 to 200,000, PS-35 having a weight average molecular weight of 310,000 to 390,000, PS-50 having a weight average molecular weight of 400, 000 to 600, 000, and PS-100 having a weight average molecular weight of 800,000 to 1,200,000, manufactured by Tosoh Organic Chemical Co., LTd), styrenesulfonic acid-methacrylic acid copolymers (e.g., MA-2005L having a weight average molecular weight of 4200 manufactured by Tosoh Organic Chemical Co., LTd), styrenesulfonic acid-styrene copolymers

(e.g., ST-3510 having a weight average molecular weight of 74,000, and ST-5005 having a weight average molecular weight of 24,000, manufactured by Tosoh Organic Chemical Co., LTd).

The weight average molecular weight of the polymer or copolymer of the component (A) (as measured by gel filtration chromatography (in terms of polyethylene glycol)) can be controlled within the range from 1,000 to 1,400,000 by employing suitable polymerization conditions. In the present invention, from the viewpoint of sprayability and applicability of the hair cosmetic composition, the weight average molecular weight is preferably from 5, 000 to 1, 300, 000, more preferably from 30,000 to 1,200,000, and more preferably from 60,000 to 800,000.

The content of the polymer or copolymer of the component (A) in the hair cosmetic composition is preferably from 0.01 to 20% by mass, more preferably from 0.1 to 10% by mass, and more preferably from 0.5 to 7% by mass, from the viewpoint of providing high hair styling property and preventing stiffening.

[(B): Cyclodextrin]

The hair cosmetic composition of the present invention comprises cyclodextrin as component (B). Cyclodextrin is not particularly limited as long as it is water-soluble, and preferably has solubility of 0.1 g or more, more preferably 5 g or more, and more preferably 10 g or more in 100 mL of water at room temperature. The cyclodextrin is preferably substituted or unsubstituted α-cyclodextrin, substituted or unsubstituted β-cyclodextrin, or substituted or unsubstituted γ-cyclodextrin. Specific examples include α-cyclodextrin, β-cyclodextrin, γ-cyclodextrin, maltosyl-β-cyclodextrin, hydroxyethyl-β-cyclodextrin, hydroxypropyl-β-cyclodextrin, hydroxypropyl-γ-cyclodextrin, monochlorotriazinyl-β-cyclodextrin, and methyl-β-cyclodextrin. The cyclodextrin of the component (B) may be used alone or in combination of two or more of them. Among them, substituted or unsubstituted α-cyclodextrin is more preferred from the viewpoints of prevention of hair dangling, retention of hair setting after application, and water solubility.

The content of the cyclodextrin of the component (B) in the hair cosmetic composition is preferably from 0 .1 to 30% by mass, more preferably from 0.2 to 25% by mass, more preferably from 0.3 to 20% by mass, yet more preferably from 0.4 to 20% by mass, yet more preferably from 0.5 to 15% by mass, yet more preferably from 1.1 to 15% by mass, and yet more preferably from 3 to 15% by mass, from the viewpoint of preventing hair dangling, retaining hair setting after application.

The content mass ratio of the component (A) and component (B) (component (A) : component (B)) is preferably from 1 : 1 to 1 : 1300, more preferably from 1 : 1 to 1:1000, more preferably from 1 : 1 to 1 : 500, and yet more preferably from 1:1 to 1:10, from the viewpoint of preventing hair dangling after application, and obtaining preferable hair washability while maintaining high moisture resistance.

[(C): Water]

The hair cosmetic composition of the present invention comprises 50 to 95% by mass of water as component (C). The content of water as component (C) is preferably from 60 to 95% by mass, more preferably from 70 to 95% by mass, and more preferably from 80 to 95% by mass, from the viewpoint of reducing the usage of the organic solvent.

[(D): Aromatic sulfone compound]

The hair cosmetic composition of the present invention may further comprise the aromatic sulfone compound represented by the general formula (3) as component (D):

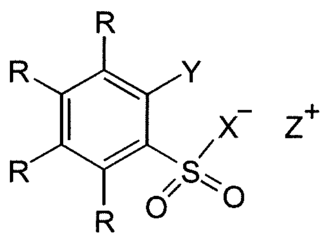

(3)

wherein R may be the same or different and independently represent a hydrogen atom or a monovalent hydrocarbon group, or adjacent two Rs may be combined to form a saturated or unsaturated divalent hydrocarbon group; X represents an oxygen atom or a nitrogen atom, Y represents a hydrogen atom when X is an oxygen atom, or Y represents a carbonyl group bound to X when X is a nitrogen atom; $Z^+$ represents a monovalent cation.

When the R is the monovalent hydrocarbon group, examples thereof include an alkyl group, an aryl group, and an aralkyl group. Among them, an alkyl group having 1 to 8 (preferably 1 to 4) carbon atoms and an aryl or aralkyl group

having 6 to 10 (preferably 6 to 8) carbon atoms are preferred, and specific examples include a methyl group, an ethyl group, a propyl group, an isopropyl group, a butyl group, a t-butyl group, a phenyl group, and a benzyl group.

Examples of the saturated or unsaturated divalent hydrocarbon group formed by the combination of adjacent two Rs include an alkylene group and an alkylidene group. Among them, an alkylene or alkylidene group having 2 to 6 (preferably 2 to 4) carbon atoms is preferred, and examples thereof include an ethylene group, an ethylidene group, a vinylene group, a trimethylene group, an isopropylidene group, a 1-propenylene group, a tetramethylene group, a 2-methyltrimethylene group, a 1-methyltrimethylene group, a 2-propenylene group, a 2-butenylene group, and a buta-1,3-diene-1,4-diyl. Specifically, when adjacent two Rs are combined to form a buta-1,3-diene-1,4-diyl, tetramethylene group, a 1-propenylene group, a 2-propenylene group, or a trimethylene group, the aromatic sulfone compound to be formed is naphthalenesulfonic acid, tetrahydronaphthalenesulfonic acid, indenesulfonic acid, or indanesulfonic acid, respectively. Among them, naphthalenesulfonic acid is preferred.

When X is an oxygen atom, Y represents a hydrogen atom, and it is preferred that R be alkyl groups, or adjacent two Rs be combined to form an alkylidene group. Alternatively, when X is a nitrogen atom, Y represents a carbonyl group bound to X, and R is preferably hydrogen atom.

In addition, the aromatic sulfone compound as component (D) may have any monovalent cation $Z^+$ as a counter cation. The $Z^+$ is preferably a proton, an alkali metal ion (for example, a sodium ion or a potassium ion), or an ammonium ion.

More preferred examples of the aromatic sulfone compound as component (D) include benzenesulfonic acid or a salt thereof, paratoluenesulfonic acid or a salt thereof, 2,4-dimethylbenzenesulfonic acid or a salt thereof, 2, 5-dimethylbenzenesulfonic acid or a salt thereof, naphthalenesulfonic acid or a salt thereof, and saccharin or a salt thereof. Among them, paratoluenesulfonic acid or a salt thereof, 2,4-dimethylbenzenesulfonic acid or a salt thereof, naphthalenesulfonic acid or a salt thereof, and saccharin or a salt thereof are more preferred. These compounds may be used alone or in combination of two or more of them.

The content of the aromatic sulfone compound as component (D) in the hair cosmetic composition is preferably from 0.1 to 20% by mass, more preferably from 0.5 to 10% by mass, and more preferably from 1 to 7% by mass, thereby preventing hair dangling and retaining hair setting after application, and adjusting the viscosity of the hair cosmetic composition.

[Solvent]

The solvent other than water may be contained, and examples thereof include lower alcohols (for example, ethanol, and isopropanol), and polyols such as propylene glycol, 1,3-butanediol, diethylene glycol, dipropylene glycol, and glycerol, and lactones. The content of the solvent other than water in the hair cosmetic composition is preferably from 0 to 45% by mass, more preferably from 0 to 30% by mass, more preferably from 0 to 15% by mass, more preferably from 0 to 10% by mass, more preferably from 0 to 8% by mass, and more preferably from 0 to 6% by mass, from the viewpoint of improving solubility of the components contained in the hair cosmetic composition. In addition, the content of ethanol in the hair cosmetic composition is preferably from 0 to 4% by mass, more preferably from 0 to 1% by mass, and more preferably the hair cosmetic composition is free from ethanol.

[Optional component]

The hair cosmetic composition of the present invention may comprise, in addition to the above-described components, a cosmetic oil agent without preventing the advantageous effect of the present invention. The content of the cosmetic oil agent in the hair cosmetic composition is preferably from 0.1 to 10% by mass. Examples of the cosmetic oil agent include glycerides such as castor oil, cacao oil, mink oil, avocado oil, and olive oil; waxes such as beeswax, whale wax, lanoline, and carnauba wax; higher alcohols such as cetyl alcohol, oleyl alcohol, hexadecyl alcohol, lauryl alcohol, stearyl alcohol, isostearyl alcohol, and 2-octyl dodecanol; esters such as isopropyl myristate, hexyl laurate, cetyl lactate, propylene glycol monostearate, oleyl oleate, hexadecyl 2-ethyl hexanoate, and octyldodecyl myristate; hydrocarbon oils such as liquid paraffin, vaseline, squalane, and hydrogenated polyisobutene; silicone derivatives such as dimethylpolysiloxane, methylphenyl polysiloxane, polyether-modified silicone oil, epoxy-modified silicone oil, aminomodified silicone oil, and alkyl-modified silicone oil; and polypropylene glycol. In addition, an emulsifying agent for emulsifying and stabilizing these cosmetic oil agents may be added. The emulsifying agent may be an anionic, amphoteric, cationic, or nonionic surfactant.

The hair cosmetic composition of the present invention may further comprise a perfume and a dye for improving the commercial value, and an antiseptic and an antioxidant for preventing daily deterioration of the hair cosmetic composition, and may further comprise, as necessary, a humidity controlling agent such as glycerol or propylene glycol, a curing agent, an antistatic agent, a surfactant, an anti-foaming agent, a dispersant, a thickening agent, an ultraviolet absorber, an antioxidant, an antiseptic, a colored dye, a dye fixer, and a propellant.

[Dosage form]

The dosage form of the hair cosmetic composition of the present invention is not particularly limited, and may be in the form of a transparent liquid, a lotion, an emulsion, a mist (hair mist or hair spray), or bubbles (hair mousse). Among them, the dosage form for application is preferably a mist.

When the hair cosmetic composition of the present invention is used as an aerosol hair cosmetic composition composed of an aerosol stock solution and a propellant, the content of each component means the content in the total composition of the aerosol stock solution excluding the propellant.

[Viscosity]

The viscosity of the hair cosmetic composition of the present invention is preferably 0.1 mPa·s or more, and 100 mPa·s or less, more preferably 20 mPa·s or less, and more preferably 10 mPa·s or less, thereby causing adequate stickiness when applied to hair. When used in the form of mist, the viscosity within the above-described range allows spraying of the hair cosmetic composition in good mist form, and further allows, for example, reduction of problems such as clogging of the nozzle of the sprayer.

The viscosity is measured at 25°C after rotation at 6 rpm for 1 minute using the type B rotational viscometer (model TVB-10M), manufactured by Toki Sangyo Co., Ltd., and a rotor L/Adp No.19. The measurement is carried out in a thermostat bath at 25°C.

[PH]

The pH of the hair cosmetic composition of the present invention is measured using a glass electrode hydrogen ion concentration meter F-14 (manufactured by Horiba, Ltd.). The sample temperature is adjusted at 25°C, and the electrode is directly inserted into the sample. The pH of the hair cosmetic composition of the present invention is preferably from 2 to 9, and more preferably from 3 to 8.

[Hair styling method]

The hair cosmetic composition of the present invention is suitable as a hair styling agent. The usage of the hair styling agent, or the hair styling method may be any method as long as it includes application of the hair cosmetic composition of the present invention to hair, and arranging the hair style. The hair cosmetic composition of the present invention may be applied to wet hair or dry hair, and is preferably applied to dry hair from the viewpoint of easiness of setting.

For example, the hair cosmetic composition of the present invention may be applied after forming the hair in any hair style. The hair cosmetic composition of the present invention is preferably applied in the form of mist, thereby uniformly applying the hair cosmetic composition of the present invention without deforming the hair style. After applying the hair cosmetic composition of the present invention, the hair is air-dried. A dry film of the hair cosmetic composition of the present invention is formed between hairs by drying the hair cosmetic composition. This dry film sets the hair style, whereby the hair is formed without deformation even in high humidity.

Regarding the above-described embodiments of the present invention, preferred embodiments of the present invention are further disclosed below.

<1> A hair cosmetic composition comprising the following components (A) to (C):

(A) A polymer or copolymer having the structural units represented by the general formula (1):

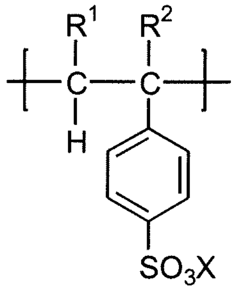

(1)

wherein $R^1$ and $R^2$ independently represent a hydrogen atom, a methyl group, or a carboxy group, X represents H, Na, K, Li, $NH_4$, or $CH_2CH_3$;
(B) cyclodextrin; and
(C) 50 to 95% by mass of water.

<2> The hair cosmetic composition of <1>, wherein the component (A) is preferably a copolymer having the structural units represented by the general formula (2):

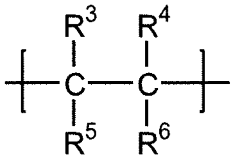
(2)

wherein $R^3$, $R^4$ and $R^5$ independently represent a hydrogen atom, a methyl group, or a carboxy group, $R^6$ represents an optionally substituted phenyl group, a substituent represented by $-COOR^7$ (wherein $R^7$ is a hydrogen atom, Na, K, Li, or an optionally substituted hydrocarbon group having 1 to 24 carbon atoms), a substituent represented by $-COR^8$ (wherein $R^8$ is an optionally substituted hydrocarbon group having 1 to 24 carbon atoms), or a substituent represented by $-COO(AO)_nR^9$ (wherein AO is an ethylene oxide group and/or a propylene oxide group, n is a numeral of 1 to 15 on average, and $R^9$ is a hydrogen atom or an optionally substituted hydrocarbon group having 1 to 5 carbon atoms.
<3> The hair cosmetic composition of <2>, wherein $R^3$, $R^4$ and $R^5$ are preferably independently a hydrogen atom or a methyl group, and $R^6$ is a phenyl group or a substituent represented by $-COOR^7$ (wherein $R^7$ is a hydrogen atom or Na).
<4> The hair cosmetic composition of any one of <1> to <3>, wherein component (A) preferably contains the structural units represented by the general formula (1) in the amount of 10 to 100% by mass, preferably 10 to 100% by mass, more preferably 20 to 100% by mass, more preferably 30 to 100% by mass, more preferably 40 to 100% by mass, more preferably 50 to 100% by mass, more preferably 60 to 100% by mass, more preferably 70 to 100% by mass, more preferably 80 to 100% by mass, and more preferably 90 to 100% by mass in one molecule.
<5> The hair cosmetic composition of any one of <1> to <4>, wherein the component (A) is preferably one or more polymers or copolymers selected from the group consisting of polystyrenesulfonic acid sodium, a styrenesulfonic acid-methacrylic acid copolymer, and a styrenesulfonic acid-styrene copolymer.
<6> The hair cosmetic composition of any one of <1> to <5>, wherein the weight average molecular weight of the component (A) is preferably from 1,000 to 1,400,000, more preferably from 5,000 to 1,300,000, more preferably from 30,000 to 1,200,000, and more preferably from 60,000 to 800,000.
<7> The hair cosmetic composition of any one of <1> to <6>, wherein the content of the component (A) in the hair cosmetic composition is preferably from 0.01 to 20% by mass, more preferably from 0.1 to 10% by mass, and more preferably from 0.5 to 7% by mass.
<8> The hair cosmetic composition of claim 1, wherein the component (B) is preferably substituted or unsubstituted $\alpha$-cyclodextrin.
<9> The hair cosmetic composition of any one of <1> to <8>, wherein the content of the component (B) in the hair cosmetic composition is preferably from 0.1 to 30% by mass, more preferably from 0.2 to 25% by mass, more preferably from 0.3 to 20% by mass, more preferably from 0.4 to 20% by mass, more preferably from 0.5 to 15% by mass, more preferably from 1.1 to 15% by mass, and more preferably from 3 to 15% by mass.
<10> The hair cosmetic composition of any one of <1> to <9>, wherein the content mass ratio of the component (A) and the component (B) ((A) : (B)) is preferably from 1 : 1 to 1 :1300, more preferably from 1 : 1 to 1 : 1000, more preferably from 1 : 1 to 1 : 500, and more preferably from 1 : 1 to 1 : 10.
<11> The hair cosmetic composition of any one of <1> to <10>, wherein the content of the component (C) in the hair cosmetic composition is preferably from 60 to 95% by mass, more preferably from 70 to 95% by mass, and more preferably from 80 to 95% by mass.
<12> The hair cosmetic composition of any one of <1> to <11>, wherein the content of ethanol in the hair cosmetic composition is preferably from 0 to 4% by mass, more preferably from 0 to 1% by mass, and more preferably the hair cosmetic composition is free from ethanol.
<13> The hair cosmetic composition of any one of <1> to <12>, which preferably further comprises the aromatic sulfone compound represented by the general formula (3) as the component (D):

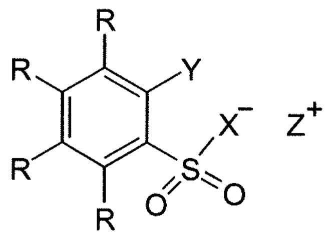

(3)

wherein R may be the same or different and independently represent a hydrogen atom or a monovalent hydrocarbon group, or adjacent two Rs are combined to form a saturated or unsaturated divalent hydrocarbon group; X represents an oxygen atom or a nitrogen atom, Y represents a hydrogen atom when X is an oxygen atom, or Y represents a carbonyl group bound to X when X is a nitrogen atom; $Z^+$ represents a monovalent cation.

<14> The hair cosmetic composition of <13>, wherein the component (D) is preferably at least one selected from the group consisting of benzenesulfonic acid, paratoluenesulfonic acid, 2,4-dimethylbenzenesulfonic acid, 2,5-dimethylbenzenesulfonic acid, naphthalenesulfonic acid, and saccharin, and salts thereof.

<15> The hair cosmetic composition of <13>, wherein the component (D) is preferably at least one selected from the group consisting of paratoluenesulfonic acid, 2,4-dimethylbenzenesulfonic acid, naphthalenesulfonic acid, saccharin, and salts thereof.

<16> The hair cosmetic composition of any one of <13> to <15>, wherein the content of the component (D) in the hair cosmetic composition is preferably from 0.1 to 20% by mass, more preferably from 0.5 to 10% by mass, and more preferably from 1 to 7% by mass.

<17> The hair cosmetic composition of any one of <1> to <16>, which is preferably applied in the form of mist.

<18> The hair cosmetic composition of any one of <1> to <17>, wherein the viscosity of the hair cosmetic composition is preferably 0.1 mPa·s or more, and preferably 100 mPa·s or less, more preferably 20 mPa·s or less, and more preferably 10 mPa·s or less.

<19> The hair cosmetic composition of any one of <1> to <18>, wherein the pH of the hair cosmetic composition is preferably from 2 to 9, and more preferably from 3 to 8.

<20> A hair styling method comprising application of the hair cosmetic composition of any one of <1> to <19> to hair.

<21> The hair styling method of <20>, wherein the hair cosmetic composition is applied to hair after setting hair to any hair style.

<22> The hair styling method of <20>or <21>, wherein the hair cosmetic composition is applied to hair in the form of a mist.

Examples

Example 1 and Comparative Example 1

Thehaircosmeticcomposition (hairmist) shown in Table 1 is prepared according to a common procedure, and the after-draw after spraying and the viscosity of the cosmetic composition were evaluated by the method described below. All the concentrations listed in the table are effective doses.

<After-draw after spraying>

Each sample was filled into a sprayer equipped with a trigger manufactured by Yoshino Kogyosho Co., Ltd. (PT-200, nozzle diameter: 0.3 mm), and the conditions of the nozzle during spraying and the presence or absence of flaking of hair after spraying were confirmed:

A: no bubble in the nozzle, and no flaking occurred; and
B: bubbles formed in the nozzle, and flaking occurred.

<Viscosity measurement>

Using type B rotational viscometer (model TVB-10M) manufactured by Toki Sangyo Co., Ltd. and a rotor L/Adp No. 19, the sample was rotated at 6 rpm speed for 1 minute at 25°C, to measure the viscosity.

[Table 1]

| (% by mass) | | Example 1 | Comparative Example 1 |
|---|---|---|---|
| (A) | Sodium polystyrene sulfonate (*1) | 1 | - |
| (A') | AMP-acrylates/C1-18 alkyl acrylate/C1-8 alkyl acrylamide copolymer (*2) | - | 1 |
| (B) | α-cyclodextrin | 5 | 5 |
| (C) | Water | Balance | Balance |
| Evaluation | After-draw after spraying | A | B |
| | Viscosity (mPa·s) | 8.5 | 3 |
| *1: PS-50(manufactured by Tosoh Organic Chemical Co., LTd)<br>*2: The remainder of PLAS CIZE L-9909B (40% by mass ethanol solution, manufactured by Goo Chemical Co., Ltd.) after evaporation of ethanol as the solvent. | | | |

Examples 2 to 20 and Comparative Examples 2 to 16

The hair cosmetic compositions (hair mists) shown in Tables 2 to 4 were prepared according to a common procedure, and "effect of preventing hair dangling after application", "hair set retentivity (in high humidity)", "washability", and "sprayability" were evaluated according to the methods described below. All the concentrations listed in the table are effective doses.

<Effect of preventing hair dangling after application>

A hair bundle having a length of 25.5 cm and a weight of 3 g were moistened with water, wound around a rod (pipe) having a diameter of 2.2 cm, and air-dried (allowed to stand at room temperature for 24 hours or longer). After drying, the rod was removed, and the apparent length (L) of the hair bundle (unit: cm) was measured. Subsequently, 0.6 g of each sample was uniformly applied to the hair bundle using a sprayer equipped with a trigger manufactured by Yoshino Kogyosho Co., Ltd. (PT-200, nozzle diameter: 0.3 mm), the hair bundle was hung at a temperature of 25°C and a humidity of 40%, and the apparent length ($L_0$) of the hair bundle (unit: cm) was measured after 30 minutes.

The rate of prevention of hair dangling was determined from these measurements by the following formula:

$$\text{The rate of prevention of hair dangling (\%)} = [(25.5 - L_0) / (25.5 - L)] \times 100$$

The closer the value is to 100, the higher the effect of preventing hair dangling is.

<Set retentivity (in high humidity)>

0.75 g of each sample was uniformly applied to a hair bundle having a length of 26 cm and a weight of 1 g using a dropper, the hair bundle was wound around a rod (pipe) having a diameter of 2.2 cm, and allowed to stand in a temperature-controlled room at 40°C for 6 hours for completely drying. After drying, the rod was removed, and the apparent length (L) of the hair bundle (unit: cm) was measured. Subsequently, the hair bundle was suspended in a constant temperature and humidity chamber at a temperature of 25°C and a humidity of 90%, taken out after 2 hours, and the apparent length ($L_0$) of the hair bundle (unit: cm) was measured again. The hair set retentivity was determined from these measurements using the following formula:

$$\text{Hair set retentivity (\%)} = [(26 - L_0) / (26 - L)] \times 100$$

The closer the value to 100, the stronger the hair set retentivity.

<Washability>

0.75 g of each sample was uniformly applied using a dropper to a hair bundle having a length of 26 cm and a weight of 1 g, the hair bundle was wound around a rod (pipe) having a diameter of 2.2 cm, and allowed to stand in a temperature-controlled room at 40°C for 6 hours for completely drying. After drying, the rod was removed, and the hair bundle was pinched between fingers, and rinsed with running water at a water temperature of 38°C and a flow rate of 6 L/minute while combing the hair bundle from the top to the bottom at a speed of 1 time/second.

The washability was evaluated by the number of squeezing until the sample was washed off. The hair bundle rinsed by the above-described method was naturally dried, and then lightly squeezed with fingers. The sample was regarded as having been washed off when the dry hair bundle was easily loosened:

A: the sample was washed off by squeezing less than 15 times;
B: the sample was washed off by squeezing 15 times or more and less than 30 times; and
C: the sample was not washed off by even squeezing 30 times or more.

<Sprayability>

Each sample was charged into a sprayer equipped with a trigger manufactured by Yoshino Kogyosho Co., Ltd. (PT-200, nozzle diameter: 0.3 mm), and the conditions of mist during spraying was observed:

A: sprayed in mist form (very good sprayability);
B: sprayed in the form of coarse mist (good sprayability); and
C: linearly ejected without forming mist, or not ejected (poor sprayability).

[0080]

[Table 2]

| (% by mass) | | Example | | | | | | | | | Comparative Example | | | | | | | | |
|---|---|---|---|---|---|---|---|---|---|---|---|---|---|---|---|---|---|---|---|
| | | 2 | 3 | 4 | 5 | 6 | 7 | 8 | 9 | 10 | 2 | 3 | 4 | 5 | 6 | 7 | 8 | 9 | 10 |
| (A) | Sodium polystyrenesulfonate (*3) | 5 | - | - | - | - | - | - | - | - | 5 | - | - | - | - | - | - | - | - |
| | Sodium polystyrenesulfonate (*4) | - | 5 | - | - | - | - | - | - | - | - | 5 | - | - | - | - | - | - | - |
| | Sodium polystyrenesulfonate (*5) | - | - | 1 | - | - | - | - | - | - | - | - | 1 | - | - | - | - | - | - |
| | Sodium polystyrenesulfonate (*6) | - | - | - | 1 | - | - | - | - | - | - | - | - | 1 | - | - | - | - | - |
| | Sodium polystyrenesulfonate (*1) | - | - | - | - | 1 | - | - | - | - | - | - | - | - | 1 | - | - | - | - |
| | Sodium polystyrenesulfonate (*7) | - | - | - | - | - | 1 | - | - | - | - | - | - | - | - | 1 | - | - | - |
| | Styrenesulfonic acid-methacrylic acid copolymer(*8) | - | - | - | - | - | - | 5 | - | - | - | - | - | - | - | - | 5 | - | - |
| | Styrenesulfonic acid-styrene copolymer(*9) | - | - | - | - | - | - | - | 5 | - | - | - | - | - | - | - | - | 5 | - |
| | Styrenesulfonic acid-styrene copolymer(*10) | - | - | - | - | - | - | - | - | 5 | - | - | - | - | - | - | - | - | 5 |
| (B) | α-cyclodextrin | - | - | 5 | 5 | 5 | 5 | 5 | 5 | 5 | - | - | - | - | - | - | - | - | - |
| | γ-cyclodextrin | 5 | 5 | - | - | - | - | - | - | - | - | - | - | - | - | - | - | - | - |
| (C) | Water | balance | balance | balance | balance | balance | balance | balance | balance | balance | balance | balance | balance | balance | balance | balance | balance | balance | balance |
| Evaluation | Rate of prevention of hair dangling after application | 80 | 80 | 80 | 80 | 80 | 80 | 80 | 80 | 80 | 58 | 58 | 60 | 60 | 65 | 65 | 58 | 58 | 58 |
| | Set retentivity (25°C, 90%RH) | 75 | 80 | 82 | 84 | 84 | 84 | 75 | 85 | 85 | 45 | 50 | 50 | 55 | 58 | 58 | 25 | 58 | 58 |
| | Hair washability | A | A | A | A | A | A | B | B | B | A | A | A | A | A | A | B | B | B |
| | Sprayability | A | A | A | A | A | A | A | A | A | A | A | A | A | A | A | A | A | A |

*3:PS-1,*4:PS-5,*5:PS-15,*6:PS-35,*7:PS-100,*8:MA-2005L,*9:ST-3510,*10:ST-5005(all manufactured by Tosoh Organic Chemical Co., LTd)

[Table 3]

| (% by mass) | | Example | | | | | | | | | |
|---|---|---|---|---|---|---|---|---|---|---|---|
| | | 11 | 12 | 13 | 14 | 15 | 16 | 17 | 18 | 19 | 20 |
| (A') | Sodium polystyrenesulfonate (*1) | 1 | 1 | 1 | 1 | 1 | 1 | 1 | - | 5 | 5 |
| | Sodium polystyrenesulfonate (*7) | - | - | - | - | - | - | - | 1 | - | - |
| (B) | α-cyclodextrin | - | - | - | 1 | 2 | 11 | 13 | 13 | 7 | 3.5 |
| | β-cyclodextrin | 1 | - | - | - | - | - | - | - | - | - |
| | Maltosyl-β-cyclodestrin (*11) | - | 5 | - | - | - | - | - | - | - | - |
| | Mixture of cyclodextrin and maltosyl-β-cyclodextrin (*12) | - | - | 5 | - | - | - | - | - | - | - |
| (D) | Naphthalenesulfonic acid | - | - | - | - | - | - | - | - | - | 1.5 |
| (C) | Water | balance | balance | balance | balance | balance | balance | balance | balance | balance | balance |
| Evaluation | Rate of prevention of hair dangling after application | 73 | 80 | 80 | 73 | 75 | 80 | 82 | 82 | 82 | 80 |
| | Set retentivity (25°C, 90%RH) | 72 | 80 | 80 | 73 | 78 | 85 | 85 | 85 | 85 | 84 |
| | Hair washability | A | A | A | A | A | A | A | B | B | A |
| | Sprayability | A | A | A | A | A | A | A | B | B | A |
| *11:G2-β-CD (manufactured by Pearl Ace Corporation)<br>*12:ISOELITE P(manufactured by Pearl Ace Corporation) | | | | | | | | | | | |

[Table 4]

| (% by mass) | | Comparative Example | | | | | |
|---|---|---|---|---|---|---|---|
| | | 11 | 12 | 13 | 14 | 15 | 16 |
| (A') | AMP-acrylates/C1-18 alkyl acrylate/C1-8 alkyl acrylamide copolymer (*2) | 1 | - | - | - | - | - |
| | Vinyl pyrrolidone-vinyl acetate copolymer (*13) | - | 1 | 5 | 5 | 5 | 5 |
| (B) | α-cyclodextrin | 8 | 8 | - | 1 | 3 | 5 |
| (C) | Water | balance | balance | balance | balance | balance | balance |
| Evaluation | Rate of prevention of hair dangling after application | 65 | 65 | 65 | 65 | 65 | 65 |
| | Set retentivity (25°C, 90%RH) | 70 | 65 | 19 | 27 | 35 | 35 |
| | Hair washability | C | A | B | B | B | B |
| | Sprayability | A | A | A | A | A | A |
| *13: LUVISKOL VA73W(manufactured by BASF SE) | | | | | | | |

Formulas

**[0051]** The hair cosmetic composition of the present invention may be produced according to a common procedure in the dosage form shown below, and used.

| Formula 1 (hair spray) | (% by mass) |
|---|---|
| PS-50 (manufactured by Tosoh Organic Chemical Co., LTd; 21% by mass aqueous solution) | 23.8 |
| α-cyclodextrin | 5.0 |
| Dimethyl ether (DME) | 10 |
| Purified water | balance |

| Formula 2 (hair spray) | (% by mass) |
|---|---|
| PS-50 (manufactured by Tosoh Organic Chemical Co. , LTd; 21% by mass aqueous solution) | 23.8 |
| α-cyclodextrin | 5.0 |
| 1,1-difluoroethane (hydrofluorocarbon 152A) | 10 |
| Purified water | balance |

| Formula 3 (hair spray) | (% by mass) |
|---|---|
| PS-100(manufactured by Tosoh Organic Chemical Co., LTd; 21% by mass aqueous solution) | 23.8 |
| γ-cyclodextrin | 7.0 |
| Dimethyl ether (DME) | 10 |
| Purified water | balance |

| Formula 4 (hair spray) | (% by mass) |
|---|---|
| PS-50 (manufactured by Tosoh Organic Chemical Co. LTd; 21% by mass aqueous solution) | 4.8 |
| α-cyclodextrin | 3.5 |
| Sodium β-naphthalenesulfonate | 1.5 |

(continued)

| Formula 4 (hair spray) | (% by mass) |
|---|---|
| Dimethyl ether (DME) | 10 |
| Purified water | balance |

| Formula 5 (hair spray) | (% by mass) |
|---|---|
| ST5005 (manufactured by Tosoh Organic Chemical Co., LTd; 21% by mass aqueous solution) | 4.8 |
| α-cyclodextrin | 3.5 |
| Sodium β-naphthalenesulfonate | 1.5 |
| 1,1-difluoroethane (hydrofluorocarbon 152A) | 10 |
| Purified water | balance |

| Formula 6 (hair mist) | (% by mass) |
|---|---|
| PS-35 (manufactured by Tosoh Organic Chemical Co., LTd; 21% by mass aqueous solution) | 4.8 |
| α-cyclodextrin | 3.5 |
| Sodium β-naphthalenesulfonate | 1.5 |
| Purified water | balance |

| Formula 7 (hair mist) | (% by mass) |
|---|---|
| PS-5 (manufactured by Tosoh Organic Chemical Co. , LTd; 21% by mass aqueous solution) | 4.8 |
| α-cyclodextrin | 3.5 |
| Sodium β-naphthalenesulfonate | 1.5 |
| Purified water | balance |

## Claims

1. A hair cosmetic composition comprising following components (A) to (C):

   (A) A polymer or copolymer having structural units represented by the general formula (1):

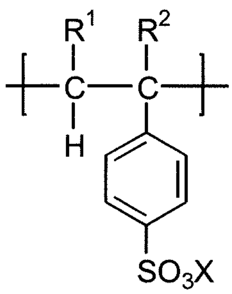

(1)

   wherein $R^1$ and $R^2$ independently represent a hydrogen atom, a methyl group, or a carboxy group, X represents H, Na, K, Li, $NH_4$, or $CH_2CH_3$;
   (B) cyclodextrin; and
   (C) 50 to 95% by mass of water.

2. The hair cosmetic composition according to claim 1, wherein the component (B) is α-cyclodextrin.

3. The hair cosmetic composition according to claim 1 or 2, which is applied in the form of a mist.

4. The hair cosmetic composition according to any one of claims 1 to 3, wherein the content of the component (A) in the hair cosmetic composition is from 0.01 to 20% by mass.

5. The hair cosmetic composition according to any one of claims 1 to 4, wherein the weight average molecular weight of the component (A) is from 1,000 to 1,400,000.

6. The hair cosmetic composition according to any one of claims 1 to 5, wherein the content of the component (B) in the hair cosmetic composition is from 0.1 to 30% by mass.

7. The hair cosmetic composition according to any one of claims 1 to 5, wherein the content of the component (B) in the hair cosmetic composition is from 3 to 15% by mass.

8. The hair cosmetic composition according to any one of claims 1 to 7, wherein the content mass ratio between the component (A) and component (B) ((A) : (B)) is from 1 : 1 to 1 : 1300.

9. The hair cosmetic composition according to any one of claims 1 to 8, which further comprises an aromatic sulfone compound represented by the general formula (3) as the component (D):

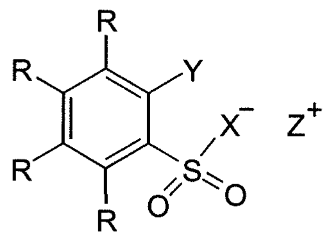

(3)

wherein R may be the same or different and independently represent a hydrogen atom or a monovalent hydrocarbon group, or adjacent two Rs are combined to form a saturated or unsaturated divalent hydrocarbon group; X represents an oxygen atom or a nitrogen atom, Y represents a hydrogen atom when X is an oxygen atom, or Y represents a carbonyl group bound to X when X is a nitrogen atom; and $Z^+$ represents a monovalent cation.

10. A hair styling method comprising application of the hair cosmetic composition according to any one of claims 1 to 9 in the form of a mist to hair.

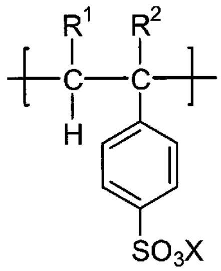

(1)

## INTERNATIONAL SEARCH REPORT

International application No. PCT/JP2013/073252

A. CLASSIFICATION OF SUBJECT MATTER
*A61K8/81*(2006.01)i, *A61K8/02*(2006.01)i, *A61K8/46*(2006.01)i, *A61K8/73*(2006.01)i, *A61Q5/06*(2006.01)i

According to International Patent Classification (IPC) or to both national classification and IPC

B. FIELDS SEARCHED

Minimum documentation searched (classification system followed by classification symbols)
A61K8/81, A61K8/02, A61K8/46, A61K8/73, A61Q5/06

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 56-166109 A (Kao Soap Co., Ltd.), 21 December 1981 (21.12.1981), claims; page 1, right column, lines 11, 12; page 2, upper left column, lines 17 to 20; example 3 (Family: none) | 1-10 |
| Y | JP 49-066836 A (National Starch and Chemical Corp.), 28 June 1974 (28.06.1974), claims; page 4, upper left column, lines 9 to 12; lower left column, lines 3 to 9; examples & GB 1439669 A & DE 2340590 A1 & FR 2198729 A1 & NL 7311607 A | 1-10 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19 November, 2013 (19.11.13) | 26 November, 2013 (26.11.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

INTERNATIONAL SEARCH REPORT

International application No. PCT/JP2013/073252

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2005-120045 A (Dai-Ichi Kogyo Seiyaku Co., Ltd.), 12 May 2005 (12.05.2005), claims; paragraphs [0009] to [0012], [0015], [0024]; examples & KR 10-2005-0037339 A & TW 284044 B | 1-10 |
| Y | WO 2002/038113 A1 (UNILEVER PLC), 16 May 2002 (16.05.2002), claims; page 4, line 25 to page 5, line 6; page 10, lines 7 to 22; examples & US 2002/0090348 A1 | 1-10 |
| Y | JP 2010-065022 A (Kao Corp.), 25 March 2010 (25.03.2010), scope of claims; claim 5; paragraphs [0007], [0028], [0058], [0095] & WO 2010/018668 A1 & EP 2314278 A1 & US 2011/0150804 A1 & CN 102119020 A | 9 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 200238113 A
- JP 2005120045 A